# EUROPEAN PATENT APPLICATION

(11) **EP 0 533 352 A2**
(43) Date of publication of application: **24.03.1993**
(21) Application number: 92307504.8
(22) Date of filing: 17.08.1992
(51) Int. Cl.: A61K 31/00, A61K 31/19, A61K 31/66, A61K 31/185

(54) **Gaba receptor agonists to treat bladder instability**

(30) Priority: 16.08.1991 GB 9117716
(71) Applicant: LYNXVALE LIMITED, Cambridge CB2 1TS (GB)
(72) Inventor: Ferguson, Douglas Robertson, Cambridge CB1 4RW (GB)
(74) Representative: Armitage, Ian Michael

(57) **Abstract**

Agonists of GABA_{A} and/or GABA_{B} recptors which are sufficiently water soluble that they enter the central nervous system (CNS) in no more than minor amounts may be used in the treatment of urinary bladder instability without the side effects of existing drugs which affect the CNS.

## Description

This invention relates to GABA (gamma amino butyric acid) receptor agonists, in particular GABA derivatives or analogues, and to such compounds for therapeutic use, particularly in bladder control.

Urinary bladder instability is a common sequel to prostatic enlargement and is relieved only in about two thirds of patients by transurethal resection of the prostate. The remainder suffer urgency of micturition, nocturia, frequency and sometimes incontinence. It has recently become clear the prostatic obstruction enhances the rate of loss bladder autonomic nerves which occurs normally with advancing age. If loss of inhibitory nerves exceeded that of excitatory nerves, one could predict that the bladder may become hyperexcitable.

It has been known for some time that non-adrenergic, non-cholinergic neurotransmission (NANC) occurs in the urinary bladder. Purinergic transmission can account for up to 60% of detrusor contraction in some species although its importance in human neurotransmission is uncertain. More recently, it has becomes clear that there are important inhibitory mechanisms in the bladder.

The amino acid, gamma-amino-butyric acid (GABA), is an established inhibitory neurotransmitter in the CNS and it has recently been identified in the urinary bladder (Kusunoki et al, 1984). GABA acts on two classes of receptors; the GABA_{A} and GABA_{B} receptor. These two receptors can be distinguished by their selective agonist and antagonists. Activation of the GABA_{A} receptor results in the entry of Cl⁻ ions into the nerves whereas activation of the GABA_{B} receptor is thought to affect the K⁺ or Ca⁺⁺ channels on nerves or smooth muscle (Bowery, 1989). GABA has been shown to inhibit nerve mediated contraction of detrusor smooth muscle strips by two groups of workers but the mechanism of action is still unclear.

The early experiments on the guinea-pig urinary bladder indicated that the actions of GABA were mediated through the GABA_{A} receptor (Taniyama et al, 1983; Kusunoki et al, 1984). However, Maggi et al (1985a,1985b) considered the major inhibitory actions to be mediated through GABA_{B} receptors.

Erdo et al (1989) used [³H]-muscimol to identify the GABA_{A} receptor sites in the guinea-pig bladder and interestingly found a large number of receptors in the bladder base and urethra, with no specific binding in the body of the bladder. However, measurements of tissue levels of GABA in guinea-pig bladder by Kusunoki et al (1984) found the highest concentration in the body of the bladder.

Attempts to localise the tissue site of action of GABA in the bladder have proved equally confusing. Kusunoki et al (1984) attributed the inhibitory actions of GABA to its capacity to reduce acetylcholine release from postganglionic parasympathetic nerve fibres, through GABA_{A} receptors. Maggi et al, (1985a, 1985b) considered on the other hand that these inhibitory pre-synaptic receptors were of the GABA_{B} type and that stimulation of GABA_{A} receptors potentiated muscular contractions. These authors also showed that GABA caused inhibition of ACh induced muscle contraction in guinea-pig bladder but they concluded that this action was mediated through some neurogenic mechanism.

In the presence of these uncertainties of the mechanism of action of GABA, the effects of GABA on nerve mediated contractions of rabbit and human urinary bladder strips were investigated.

As a result the present invention therefore comtemplates a new drug or class of drugs for use in bladder instability. Our experiments have shown the gamma amino butyric acid (GABA) is an inhibitory neurotransmitter in both rabbit and human bladders. GABA actions occur at three levels, the central nervous system, on peripheral autonomic nerves and directly on smooth muscle cells of the detrusor muscle (this is the muscle responsible for bladder emptying). The latter two effects could be produced by a water soluble GABA-like drug which by virtue of its water solubility would be unable to enter the central nervous system (including the spinal cord).

There are already drugs which act either directly on GABA receptors or to potentiate the actions of GABA. Examples are baclofen end benzodiazepine minor tranquilizers such as diazepam. Unfortunately both types of drugs have severe disadvantages through actions both inside and outside the central nervous system, particularly the production of drug dependence.

Disadvantages of present GABA agonists are as follows. GABA_{A} agonists or potentiators such as benzodiazepines and barbiturates are relatively ineffective on the bladder, but on prolonged use lead to psychological dependence as a consequence of the anti-anxiety actions. They interact very powerfully with other centrally acting drugs to produce drowsiness, lack of coordination and disorientation especially in the elderly. GABA_{B} agonists have even more side effects. For example, baclofen causes drowsiness, insomnia, dizziness, weakness and mental confusion, and very severe physical withdrawal reactions when withdrawn suddenly, suggesting that it too causes dependence.

Even a GABA_{B} agonist which does not enter the brain might affect the following systems where GABA_{B} functions are known to be important. GABA_{B} stimulation inhibits gastric motility, relaxes the bronchioles, reduces vascular tone, stimulates the oviduct and uterus, contracts the gall bladder, stimulates release of insulin, growth hormone and glucagon. It also affects ganglionic and dorsal root nerve transmission (Bowery, 1989).

Pharmacological actions of drugs presently in widespread clinical use for excitable bladders are either muscarinic or calcium channel blockers or both e.g. terodiline. These are frequently ineffective, may impede normal bladder emptying, and as a result of their non-specific properties frequently have very unpleasant side effects on other systems. In fact their use is illogical as most patients have excitatory nerve loss too, so the drugs may reduce the capacity of the bladder to contract normally, i.e. when bladder filling is complete and may even lead to acute retention of urine.

Miscellaneous drugs affecting GABA function include progabide, a GABA prodrug, and a series, of GABA transaminase inhibitors such as sodium valproate. Haubensack (1977) and Taylor and Bates (1979) published on baclofen in the bladder, but it has drawbacks as described herein.

The solution contemplated by the present invention is to target the drug very specifically to the bladder. This may be done by using a compound too water soluble to enter the central nervous system, which will be excreted in high concentration in urine and therefore have a local bladder action.

According to one aspect of the present invention there is provided a GABA receptor agonist for pharmaceutical use, in particular for control of the urinary bladder, the differential solubility of said agonist in lipid and in water being such that, in use, not more than a minor proportion of said agonist enters the central nervous system (CNS).

Preferably, the differential lipid/water solubility of said receptor agonist is chosen such that at any time after administration the concentration of the agonist in the CNS is not more than one tenth that in the general circulation, preferably not more than one twentieth of that in general circulation, and especially not more than one hundredth of that in the general circulation. The differential distribution of an agonist in the CNS and general circulation may be determined at autopsy in animal experiments.

The ability of any compound to enter the central nervous system is related to its affinity for lipid membranes; the higher the affinity for such membranes the more rapidly a compound passes into the CNS and the higher will be the concentration achieved in the CNS relative to that in the general circulation. The lipid/water partition coefficient gives a good indication of a compound's affinity for lipid and may be determined by partitioning a compound under investigation between olive oil and an aqueous phase adjusted to physiological pH. The affinity may also be estimated by determining the partitioning between a lipid solvent such as chloroform or octanol and water. The receptor agonists of the present invention are preferably less soluble in any or all of olive oil, chloroform and octanol than they are in water. For instance, the partition coefficient between olive oil, or octanol or chloroform and water is preferably less then 1:3 and, particularly preferably less than 1:5.

Preferred agonists are less soluble in any of olive oil, octanol or chloroform than is baclofen at the same pH.

As used herein the term "GABA receptor agonist" intends a compound which is able to interact with either the GABA_{A} or GABA_{B} receptor or both to initiate a reaction qualitatively similar to that produced by interaction of GABA with the same receptor or receptors.

Suitable GABA receptor agonists of the present invention may be GABA_{A} agonists or GABA_{B} agonists but are preferably GABA_{B} agonists, or agonists of both receptors. In general, the agonists are derivatives or analogues of GABA. Analogues include compounds with similar shapes and/or polarity and/or pKa to GABA itself. Suitable derivatives include GABA prodrugs.

In a second aspect the invention provides a GABA_{A} receptor agonists for pharmaceutical use, in particular for bladder control selected from
(i) the compounds:
   β-guanidinoproprionic acid; 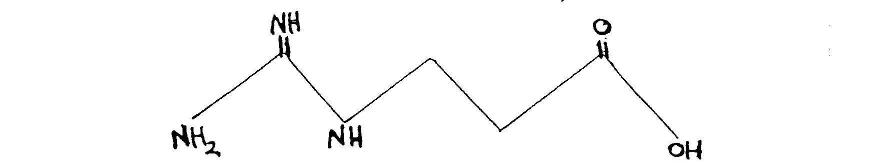 3-aminopropane sulphonic acid; 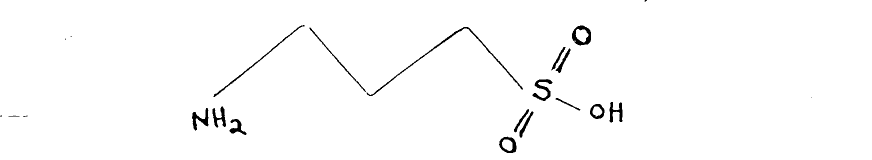 γ-amino β-hydroxybutyric acid; 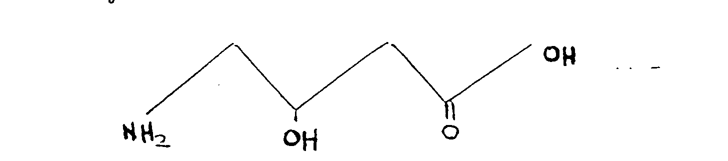 4-aminocrotonic acid; 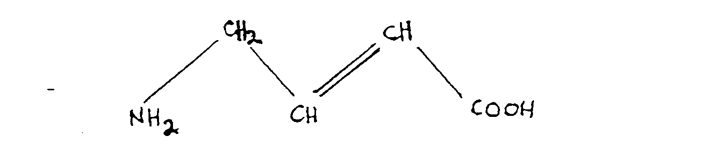 4-aminotetrolic acid; 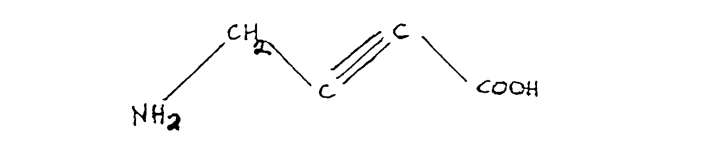 and 3-aminocyclopentane-1-carboxylic acid; 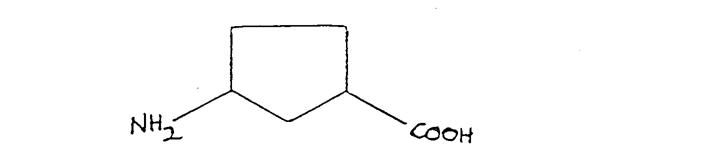
(ii) pharmaceutically acceptable salts of these compounds, and
(iii) mixtures of said compounds and/or their pharmaceutically acceptable salts.

According to a third aspect of the invention there is provided a GABA_{B} receptor agonist for pharmaceutical use, especially for bladder control, the agonist being selected from:
(i) 3-aminopropyl phosphinic acid; 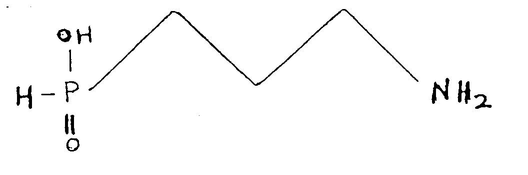 3-aminopropyl phosphonic acid; 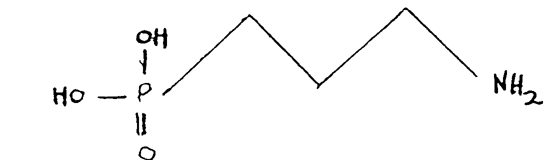
(ii) pharmaceutically acceptable salts of either of said compounds; and
(iii) mixtures of said compounds and/or their pharmaceutically acceptable salts.

According to a fourth aspect of the invention there is provided an agonist for both GABA_{A} and GABA_{B} receptors for pharmaceutical use especially for bladder control selected from:
(i) the compounds: β-hydroxy GABA, β-chloro GABA and SL-75102 of formula 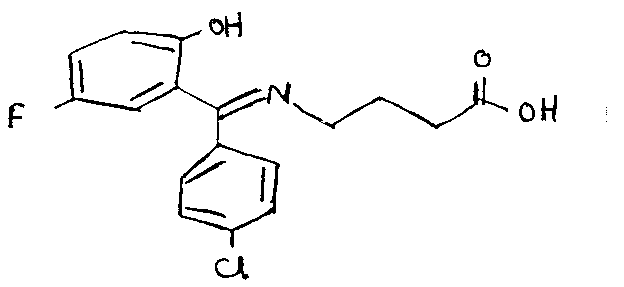
(ii) pharmaceutically acceptable salts of the said compound;
(iii) mixtures of the said compounds and/or their salts.

In a fifth aspect the invention provides a pharmaceutical composition comprising an agonist of the first, second or third aspect and a pharmaceutically acceptable excipient, diluent or carrier.

The composition of this aspect may be any conventional preparation form such as tablet, powder, capsule, solution, suspension or depot which may be prepared using conventional procedures. The composition is preferably for oral administration and might, for example, be a tablet obtained by mixing a receptor agonist with known auxiliary substances, for example, inactive diluents (e.g. lactose, calcium carbonate or calcium phosphate), binders (e.g. arabic gum, corn starch or gelatine), swelling agents (e.g. arginic acid, corn starch, pregelatined starch), sweeteners (e.g. sucrose or saccharine), flavours (e.g. peppermint, Gaultheria adenothrix oil or cherry), lubricating and wetting agents (e.g. magnesium stearate, talc or carboxymethyl cellulose).

Alternatively, but less preferably, the composition may be for local administration to the urinary bladder for instance by injection or through a urethal catheter.

In a sixth aspect the invention provides the use of an agonist of any of the first four aspects in the manufacture of a medicament for bladder control, particularly for the prevention or treatment of bladder instability.

In a still further aspect the invention provides a method of treatment of a human, or other mammalian patient, suffering from, or at risk of, bladder instability or other lack of bladder control, comprising administering to the patient an effective amount of an agonist of any one of the first four aspects of the invention.

The amount and frequency of administration will vary depending on the choice of agonist, its route of administration, age, size and condition of the patient. It is within the skills of clinician to determine an appropriate dose. In general, the daily dose may be in the range of 0.1mg to 1g. In particular, from 1-500mg, especially 1 to 50mg.

### Brief description of the drawings

Fig. 1 shows dose inhibition curves for (a) GABA (N=7)(■) and for (B)(±) baclofen (N=8)(●). Results expressed as the percentage inhibition of muscle contractions by electrical field stimulation of controls (10Hz, 0.2ms pulse width, 30V for 3s). Values are means (±S.E.M.). Computer generated best fit curves are shown.

Fig. 2 shows frequency responses curves for muscle strips with the addition of 1x10⁻⁵ GABA (a) or with the addition of 1x10⁻⁴M baclofen (b). Each point represents the mean ±S.E.M. of values. Comparisons of the values from the control values with the t test *P<0.05; **P<0.005; ***P<0.001; ****P<0.0005.

Fig. 3 shows dose inhibition curve for GABA alone (n=7)(■) with the addition of 2-hydroxysaclofen (n=8)(●) and with the addition of bicuculline (n=6)( 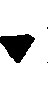 ). Values are means (±S.E.M.). Computer generated best fit curves are shown.

Fig. 4 shows concentration dependent inhibition of carbachol 5x10⁻⁶M induced muscle contractions by the addition of (1) GABA (n=10) and by (b) baclofen (n=6). Values are means (±S.E.M.). Comparison of the values from the control valves with the t test *P<0.05; **P<0.001.

### Experimental

Male Dutch rabbits weighing 800-1000g were killed and the bladder removed and placed in ice cold Krebs' solution. Muscle strips were obtained measuring approximately 8mm in length and 2mm in diameter. They were stored for a maximum of 20 hours at 4°C in oxygenated Krebs' solution before their use. The strips were suspended in a superfusion chamber (capacity 0.2ml) of the Brading and Sibley design (1983) and superfused with Krebs' solution (NaCl 124; KCl 5; MgCl₂ 1.3; NaHCO₃ 26; CaCl₂ 0.8; KH₂PO₄ 1.4 and Glucose 10 [mM] continually gassed with 95% O₂ and 5% CO₂) at 37°C and at 1ml/min. A loading tension of 0.5g was applied and the strips were allowed to equilibrate for 1 hour. The muscle strips were then stimulated with electrical field stimulation at a constant frequency to ensure that stable reproducible contractions were obtained.

Nerve mediated contractions were obtained using electrical field stimulation by means of two platinum electrodes. The stimulation parameters used (3 s trains of 30V square wave pulses with 0.2 ms pulse width in the range of 1 to 40 Hz) were shown to stimulate selectively the intramural nerves rather than the smooth muscles in the strips; this was confirmed by abolishing the contractions with the addition of tetrodotoxin (TTX) (1 x 10⁻⁷M). Frequency response curves were constructed in the range of 1 to 40 Hz with stimuli repeated at 4 min intervals and then repeated in the presence of GABA and its analogues. Dose response curves to inhibitors were constructed by using a constant frequency of 10 Hz and taking the mean of four responses at each drug concentration and expressing the result as a percentage inhibition of the control contraction. A five minute interval was allowed for each change of solution before electrical field stimulation was applied.

Carbachol mediated muscle contractions were obtained by adding the concentration of carbachol (5x10⁻⁶ M) to the superfusion chamber and exposing the tissue for ten seconds. The effects of GABA and baclofen on carbachol mediated contractions were then observed by superfusing the tissue with the concentration of the drugs dissolved in Kreb's solution.

Statistical analysis was performed using Student's "t" test. Comparisons of the dose response curves was performed by computer curve fitting using a Hill equation. The EC₅₀ (the stimulus required to produce a contraction which is 50% of the maximal response) and the maximum response was obtained from the computer estimates and compared between two groups the Student's "t" test. The curve fitting programme was no suitable for the frequency response curves and comparisons of corresponding points on the curve were made with the "t" test. Significance levels unless stated are P,0.05.

The drugs used were GABA; (±) baclofen (GABA_{B} antagonist); muscimol (GABA_{A} agonist); 2-hydroxysaclofen (GABA_{B} antagonist); bicuculline methobromide (GABA_{A} antagonist); 3-aminopropylphosphorous acid (also known as 3-aminopropylphosphinic acid) (GABA_{B} agonist) carbachol and tetrodotoxin. All the drugs were obtained from Sigma Chemicals except for 2-hydroxysaclofen, and 3-aminopropylphosphinic acid which was obtained from Tocris Neuramin.

### Effects of GABA on nerve mediated muscle contractions

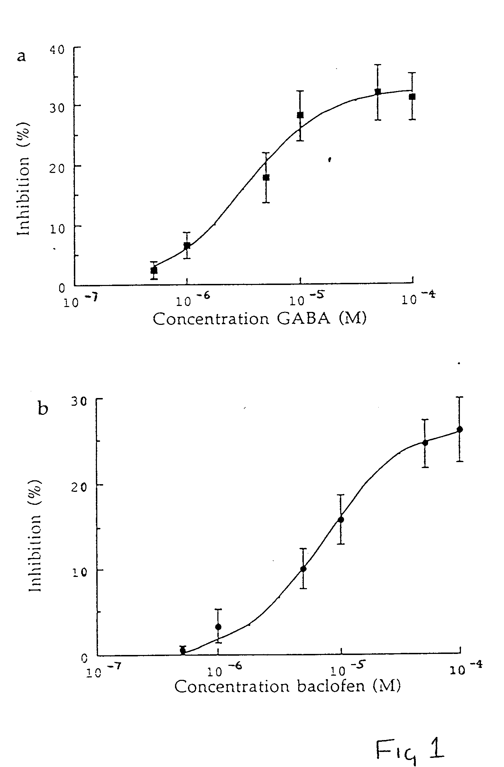

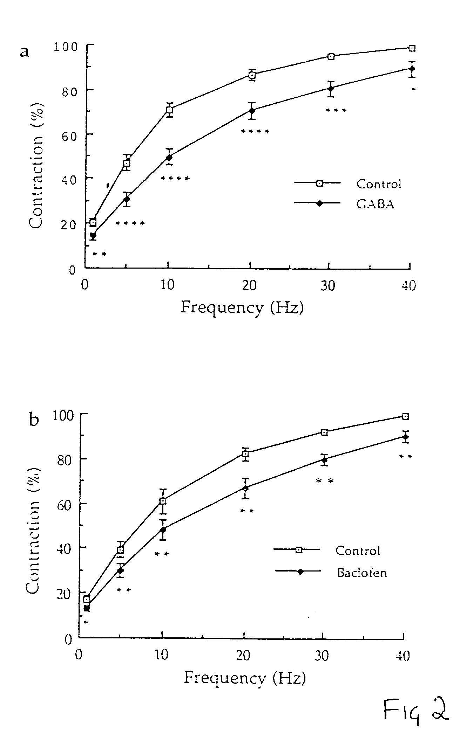

GABA produced a concentration dependent inhibition of muscle contraction induced by electrical field stimulation at Hz. The maximum inhibition was 32.5 (±1.9)% with an EC₅₀ of 3.42 (±0.69)x10⁻⁶M (n=7 strips) (Fig. 1a). GABA also caused a significant right shift of the frequency response curve (n=15) (Fig. 2a).

### Effects of GABA agonists

(±) baclofen also produced a concentration dependent inhibition with a maximum inhibition of 26.5(±1.58)% and an EC₅₀ of 7.26 (±1.2)x10⁻⁶M (n=8)(Fig. 1b), and caused a significant inhibitory effect on the frequency response curve (n=13)(Fig. 2b). Muscimol, the GABA_{A} receptor agonist, in a concentration range of 1x10⁻⁸ to 1x10⁻⁵M had no inhibitory effect (n=5).

### Effects of antagonists on GABA inhibition

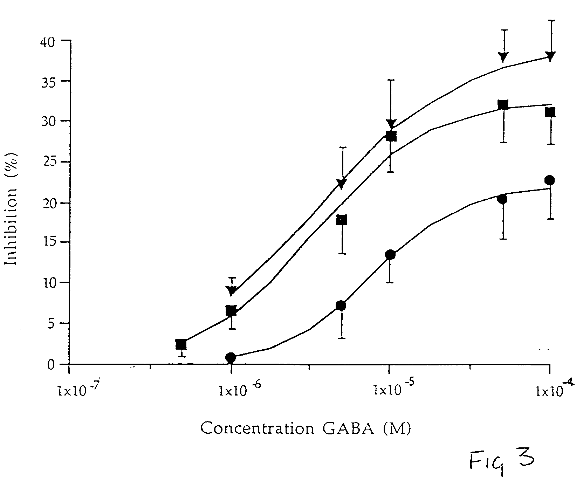

The addition of the GABA_{B} antagonist, 2-hydroxysaclofen (1 x 10⁻⁵M), caused a significant antagonism of the inhibition produced by GABA, with an increase of the EC₅₀ to 7.82 (±0.62)x10⁻⁶ µm (n=8) (P,0.0005) and no significant difference in the maximum response. The addition of biocuculline methobromide (1x10⁻⁵M) to GABA produced no significant change in the EC₅₀(3.85±0.39x10⁻⁶M) but caused a significant increase in the maximum response (P,0.0025)(n=6)(Fig.3).

### Effects on carbachol induced muscle contractions

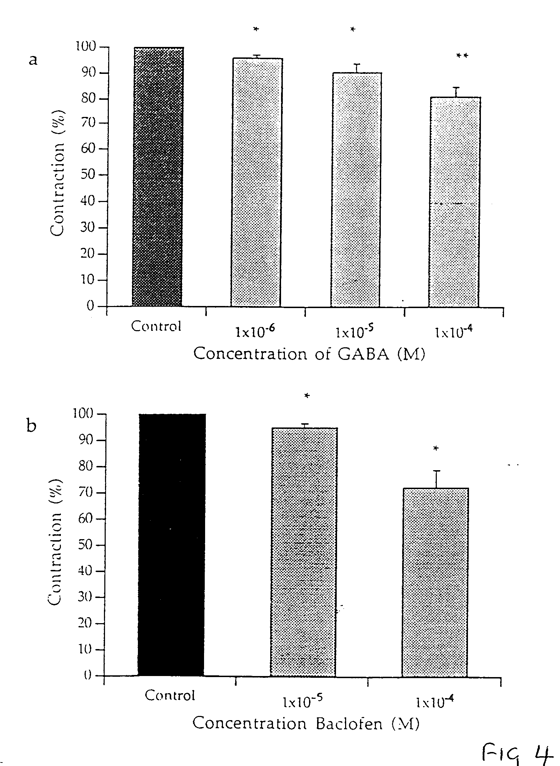

GABA produced a dose dependent inhibition of carbachol (5x10⁻⁶M) induced muscle contraction in 10 strips (Fig. 4b), however, no inhibition was seen in 6 strips. In 4 experiments , the addition of TTX (1x10⁻⁷M) did not abolish the inhibition produced by 1x10⁻⁶M dose of GABA. (±) baclofen was also able to inhibit the muscle contraction in 6 strips although the effect was not seen in 3 strips (Fig. 4b).

### Conclusions

Our findings demonstrate that the inhibitory effect of GABA on muscle strip contraction induced by electrical field stimulation of the intramural nerves is mediated by the GABA_{B} receptor. This conclusion stems from the findings that the inhibitory actions are mimicked by (±) baclofen, a GABA_{B} receptor agonist, but not by muscimol, the GABA_{A} receptor agonist. In addition the inhibitory actions can be reversed by the GABA_{B} receptor antagonist 2-hydroxy saclofen but not by bicuculline, a GABA_{A} receptor antagonist. These results support the findings of Santicioli et al (1984) that in the rabbit detrusor, the inhibitory actions of GABA are mediated by the GABA_{B} receptor.

Our findings that both GABA and baclofen inhibited carbachol induced muscle contractions and that these effects were not inhibited by TTX (1x10⁻⁷M) is best explained if GABA has a direct effect on detrusor smooth muscle cells through GABA_{B} receptors. Although the improvement seen in patients with idiopathic detrusor instability (Taylor and Bates, 1979), and with neurogenic voiding disturbances (Haubensack, 1977) following treatment, has been attributed to its actions on the spinal cord, it could equally well be explained by its action on the bladder itself. The failures of baclofen and GABA sometimes to produce direct inhibition of bladder smooth muscle cells suggest that GABA may have a more complex action on bladder function.

In summary, GABA inhibits contraction of bladder strips when they are made to contract by electrical stimulation of their autonomic nerves. This inhibition is mediated via the GABA_{B} receptors, though the experiments described above do not exclude other GABA mediated actions on bladder function.

The inventor concludes that GABA appears to exert its inhibitory actions at several levels. At the level of the smooth muscle cells it works through GABA_{B} receptors, but at the neural level GABA inhibits acetylcholine release through GABA_{A} receptors. This conclusion has led the inventor to the GABA agonists described above as the first to fourth aspects of the invention.

The properties of the ideal drug for treating bladder instability are likely to be as follows.
(a) It should be an agonist at both types of GABA receptor.
(b) It should be sufficiently water soluble in the general circulation so as not to enter the brain. The drug as administered in the gastro-intestinal tract should be well adsorbed, and as appearing in the urine within the bladder should be sufficiently well adsorbed there to act on the bladder smooth muscle GABA receptors.
(c) GABA receptors are widespread both centrally and peripherally so there appear to be advantages to be gained from targeting the drug as closely as possible.
(d) There are other possibilities, for example a reasonably water soluble GABA_{B} agonist would probably be effective provided that it were well absorbed from the gastro-intestinal tract and it penetrated the brain too poorly to have any detectable side effects.
(e) It should not be a substrate for either the high or low affinity cellular GABA uptake systems.

It is therefore possible to screen potentially useful compounds according to the foregoing criteria.

Use of smooth muscle strips from rabbit bladders stimulated to contract by electrical field stimulation is simple, accurate, reliable and relatively cheap, and would be suitable as an initial biological screening procedure.

It is also possible to demonstrate the effects of GABA agonists on release of acetylcholine from autonomic nerves, and compounds found to be active on initial screening could be tested in this way.

A third test is to perform cystometrograms on rabbits. This enables the inhibitory capability on whole animals of the prospective compounds to be monitored, and in particular their actions on the micturition cycle in whole animals to be investigated.

### Effect of 3-aminopropylphosphonous acid on nerve mediated muscle contractions

In order to confirm these conclusions preliminary experiments were performed with the water soluble GABA_{B} agonist 3-aminopropylphosphonous acid, also known as 3-aminopropylphosphinic acid, whose structure is shown below.

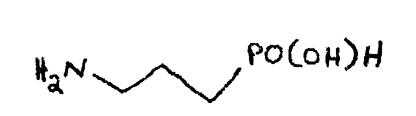

Rabbit urinary bladder smooth muscle strips were subjected to electrical stimulation as described above. the average EC₅₀ over seven experiments was found to be 250 ± 160 nm, while the maximum inhibition of contraction expressed as a percentage of the drug-free control contraction was 45 ± 18%.

These results confirm that water soluble agonists of GABA receptors are of utility in the regulation of bladder instability.

### References

Bowery, N. (1989). GABA receptors and their significance in mammalian pharmacology. Trends.Pharmacol. Sci. 10: 401-407.

Brading, A.F. and Sibley G.N.A. (1983). A superfusion apparatus to study stimulation of smooth muscle from mammalian urinary bladder. J. Physiol. (LONDON) 334, 11-12p.

Erdo, S.L., Mione, M.C., Amenta, F. and Wolff, J.R. (1989) Binding of [³H]-muscimol to GABA_{A} sites in the guinea-pig urinary bladder: biochemical assay and autorediography.Br.J.Pharmac. 96: 313-318.

Haubensack, K. (1977). A double blind trial with the antispasticity drug lioresal in 15 paraplegics with upper motor neuron lesions. Urol.Int. 32: 198-204.

Kusunoki, M., Taniyama, K. and Tanaka, C. (1984). Neuronal GABA reelease and GABA inhibition of ACh release in guinea-pig urinary bladder. Am.J.Physiol. 246: R502-R509.

Maggi, C.A., Santicioli, P. & Meil, A. (1985a). GABA_{A} and GABA_{B} receptors in detrusor strips from guinea-pig bladder dome. J.Auton.Pharmac. 5,55-64.

Maggi, C.A., Santicioli, P. and Meli, A. (1985b). Dual effect of GABA on the contractile activity of the guinea-pig isolated urinary bladder. J. Auton. Pharmac. 5:131-141.

Santicioli, P., Maggi, C.A. and Meli, A. (1984). GABA_{B} receptor mediated inhibition of field stimulation-induced contractions of rabbit bladder muscle in-vitro. J.Pharm.Pharmacol. 36:378-381.

Taniyama, K. Kusunoki, M. and Tanaka, C. (1983). Aminobutyric acid inhibits mobility of the isolated guinea-pig urinary bladder. Eur.J.Pharmacol. 89: 163-166.

Taylor, M.C. and Bates, C.P. (1979). A double-blind crossover trial of baclofen- a new treatment for the unstable bladder syndrome. Br.J.Urol. 51:504-505.

## Claims

1. A GABA (γ-amino butyric acid) receptor agonist for pharmaceutical use in mammals, the agonist having a lipid/water partition coefficient such that, in use, no more than a minor amount of the agonist enters the central nervous system (CNS) of the mammal.

2. A GABA receptor agonist according to claim 1 whose lipid/water partition coefficient is such that, in use, the concentration of the agonist in the CNS at any time is not more than one tenth that in the general circulation.

3. A GABA receptor agonist according to claim 1 or claim 2 which is less soluble in olive oil, octanol or chloroform than in any aqueous phase at physiological pH.

4. A GABA receptor agonist according to any one of the preceding claims which is a GABA_{B} receptor agonist.

5. A GABA receptor agonist according to any one of the preceding claims which is an agonist of GABA_{A} and GABA_{B} receptors.

6. A GABA receptor agonist according to any one of the preceding claims wherein the GABA receptor agonist is a GABA analogue, derivative or prodrug.

7. A GABA_{A} receptor agonist for pharmaceutical use selected from:
(i) the compounds:
β-guanidinoproprionic acid; 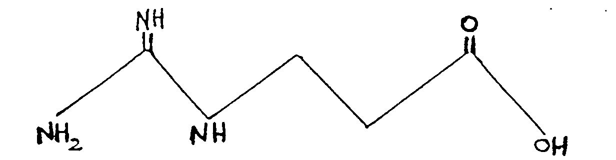 3-aminopropane sulphonic acid; 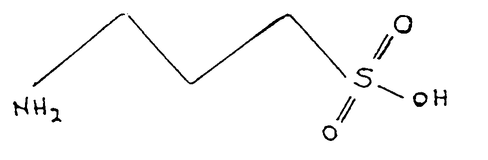 γ-amino β-hydroxybutyric acid; 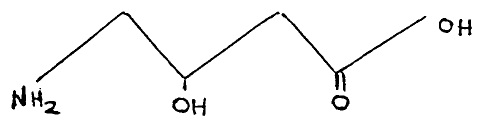 4-aminocrotonic acid; 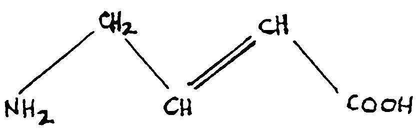 4-aminotetrolic acid; 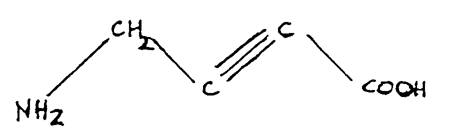 and 3-aminocyclopentane-1-carboxylic acid ; 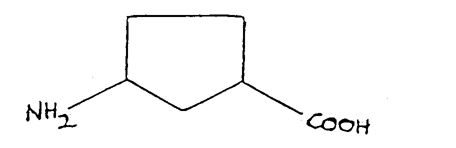
(ii) pharmaceutically acceptable salts of these compounds; and
(iii) mixtures of said compounds and/or their pharmaceutically acceptable salts.

8. A GABA_{B} receptor agonist for pharmaceutical use selected from:
(i) the compounds:
3-aminopropyl phosphinic acid ; and 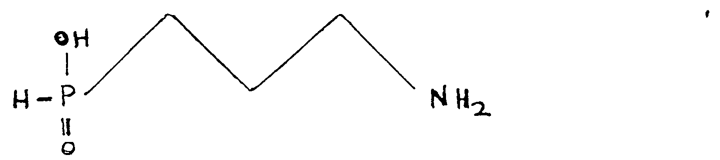 3-aminopropyl phosphonic acid ; 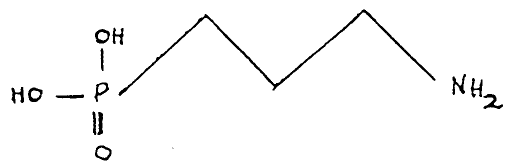
(ii) pharmaceutically acceptable salts of either of said compounds; and
(iii) mixtures of said compounds and/or their pharmaceutically acceptable salts.

9. An agonist of both GABA_{A} and GABA_{B} receptors for pharmaceutical use selected from:
(i) the compounds: β-hydroxy GABA, β-chloro GABA and SL-75102 of formula: 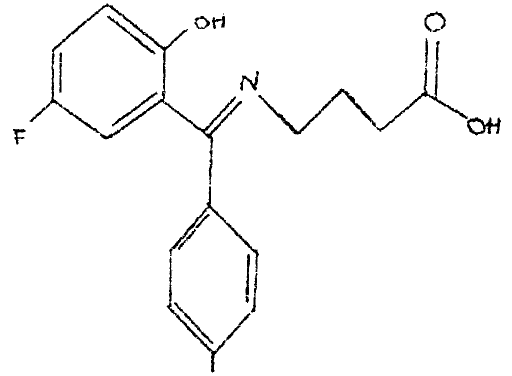
(ii) pharmaceutically acceptable salts of the said compounds;
(iii) mixtures of the said compounds and/or their salts.

10. A pharmaceutical composition comprising a GABA receptor agonist according to any one of the preceding claims and a pharmaceutically acceptable excipient, diluent or carrier.

11. Use of a GABA receptor agonist of any one of claims 1-9 in manufacture of a medicament for the prevention or treatment of bladder instability.

12. A use according to claim 11 wherein the medicament is for oral administration.

13. Use acccording to claim 11 wherein the medicament is for local administration to the bladder.

14. Use according to any one of claims 11 to 13 wherein the medicament is a dosage form arranged for administration of a daily dose of 0.1mg to 1g of the GABA receptor agonist.
